# EUROPÄISCHE PATENTANMELDUNG

(11) **EP 4 368 587 A1**
(43) Veröffentlichungstag der Anmeldung: **15.05.2024**
(21) Anmeldenummer: 22206185.5
(22) Anmeldetag: 08.11.2022
(51) Int. Cl.: C03B 32/00, C03C 10/00, C03B 27/012

(54) **VERFAHREN ZUR HERSTELLUNG EINES MEHRFARBIGEN GLASKERAMIK-ROHLINGS FÜR DENTALE ZWECKE, MEHRFARBIGER GLASKERAMIK-ROHLING, SOWIE VERWENDUNG DESSELBEN**

(71) Anmelder: Ivoclar Vivadent AG, 9494 Schaan (LI)
(72) Erfinder: ARNOLD, Lars, 9475 Sevelen (CH); ENTNER, Walter, 6830 Rankweil (AT)
(74) Vertreter: Maiwald GmbH

(57) **Zusammenfassung**

Es wird ein Verfahren zur Herstellung eines mehrfarbigen Glaskeramik-Rohlings (10) für dentale Zwecke beschrieben. Der Glaskeramik-Rohling (10) wird aus wenigstens einem ersten Werkstoffpulver (18) und einem zweiten Werkstoffpulver (20) hergestellt, wobei das erste Werkstoffpulver (18) und das zweite Werkstoffpulver (20) unterschiedlich gefärbt sind und wobei wenigstens eines aus erstem Werkstoffpulver (18) und zweitem Werkstoffpulver (20) Nanopartikel (14) und/oder Glaskeramik-Partikel (16) umfasst. Im Zuge des Verfahrens werden das erste Werkstoffpulver (18) und das zweite Werkstoffpulver (20) in eine Form (22) eingebracht, um wenigstens eine Pulvergemischansammlung (26) zu bilden. Zusätzlich wird die Pulvergemischansammlung (26) mittels Heißpressen verdichtet, um den Glaskeramik-Rohling (10) zu bilden. Außerdem wird ein mehrfarbiger Glaskeramik-Rohling (10) vorgestellt, der mittels eines solchen Verfahrens erhältlich ist. Zudem wird eine Verwendung des mehrfarbigen Glaskeramik-Rohlings (10) als Dentalmaterial beschrieben.

## Beschreibung

Die Erfindung betrifft ein Verfahren zur Herstellung eines mehrfarbigen Glaskeramik-Rohlings für dentale Zwecke.

Ferner ist die Erfindung auf einen mehrfarbigen Glaskeramik-Rohling gerichtet, der mittels eines solchen Verfahrens erhältlich ist.

Zudem betrifft die Erfindung die Verwendung eines derartigen, mehrfarbigen Glaskeramik-Rohlings als Dentalmaterial.

In diesem Zusammenhang ist es bekannt, Glaskeramik-Rohlinge in der Dentaltechnik einzusetzen. Mehrfarbige Glaskeramik-Rohlinge haben dabei den Vorteil, dass sie insbesondere im Vergleich zu einfarbigen Glaskeramik-Rohlingen die optischen Eigenschaften von natürlichem Zahnmaterial sehr gut nachahmen können. Damit eignen sich mehrfarbige Glaskeramik-Rohlinge besonders für die Herstellung von ästhetisch anspruchsvollen Dentalrestaurationen mit sehr guten optischen und mechanischen Eigenschaften. Die optischen Eigenschaften betreffen dabei nicht nur die Farbe, sondern auch die Transluzenz der Dentalrestauration.

Die Aufgabe der Erfindung besteht darin, bekannte Verfahren zur Herstellung von mehrfarbigen Glaskeramik-Rohlingen weiter zu verbessern. Es soll dabei insbesondere ein Verfahren angegeben werden, mit dem in einfacher Weise ein mehrfarbiger Glaskeramik-Rohling hergestellt werden kann, mit dem die optischen Eigenschaften von natürlichem Zahnmaterial sehr gut nachgeahmt werden können, dem in einfacher Weise maschinell die SE:TOP

Form der schließlich gewünschten Dentalrestauration gegeben werden kann und der nach der Formgebung zu einer Dentalrestauration mit ausgezeichneten mechanischen und optischen Eigenschaften umgewandelt werden kann.

Die Aufgabe wird durch ein Verfahren zur Herstellung eines mehrfarbigen Glaskeramik-Rohlings für dentale Zwecke gelöst. Der mehrfarbige Glaskeramik-Rohling wird aus wenigstens einem ersten Werkstoffpulver und einem zweiten Werkstoffpulver hergestellt, wobei das erste Werkstoffpulver und das zweite Werkstoffpulver unterschiedlich gefärbt sind. Zudem umfasst wenigstens eines aus erstem Werkstoffpulver und zweitem Werkstoffpulver Nanopartikel und/oder Glaskeramik-Partikel. Das Verfahren umfasst:
- Einbringen des ersten Werkstoffpulvers und des zweiten Werkstoffpulvers in eine Form, um wenigstens eine Pulvergemischansammlung zu bilden, und
- Verdichten der Pulvergemischansammlung mittels Heißpressen, um den Glaskeramik-Rohling zu bilden.

Das erfindungsgemäße Verfahren schließt mehrere Varianten ein.

In einer ersten Variante des erfindungsgemäßen Verfahrens werden das erste Werkstoffpulver und das zweite Werkstoffpulver jeweils trocken in die Form eingebracht. Optional bildet dabei das erste Werkstoffpulver und/oder das zweite Werkstoffpulver ein Granulat. Dabei werden das erste Werkstoffpulver und das zweite Werkstoffpulver derart in die Form dosiert, dass sich ein vorgegebener, kontinuierlicher Verlauf der optischen Eigenschaften, insbesondere ein kontinuierlicher Farbverlauf, innerhalb der Form ergibt. In dieser Variante kann zudem die durch das erste Werkstoffpulver und das zweite Werkstoffpulver gebildete Pulvergemischansammlung direkt mittels Heißpressen zum Glaskeramik-Rohling verarbeitet werden. Optional wird die Pulvergemischansammlung über den Zwischenschritt einer Wärmebehandlung zum Glaskeramik-Rohling verarbeitet. Im Zwischenschritt der Wärmebehandlung kann wenigstens ein Bestandteil der Pulvergemischansammlung, der im Glaskeramik-Rohling unerwünscht ist, thermisch entfernt werden. Beispielsweise werden in diesem Zusammenhang Bindemittel mittels der Wärmebehandlung ausgebrannt.

In einer zweiten Variante des erfindungsgemäßen Verfahrens werden das erste Werkstoffpulver und/oder das zweite Werkstoffpulver als Suspension in die Form eingebracht. Bei der Suspension handelt es sich insbesondere um eine wässrige Suspension des ersten Werkstoffpulvers und/oder eine wässrige Suspension des zweiten Werkstoffpulvers. In dieser Variante muss die Pulvergemischansammlung stets getrocknet werden, bevor sie zum Glaskeramik-Rohling weiterverarbeitet wird. Es fällt also ein Trocknungsschritt an. Die getrocknete Pulvergemischansammlung kann dann direkt zum Glaskeramik-Rohling weiterverarbeitet werden. Wie bereits im Zusammenhang mit der ersten Variante erläutert, kann auch in der zweiten Variante ein Zwischenschritt zur Herstellung eines Grünlings und/oder ein Zwischenschritt zur Herstellung eines Weißlings vorgesehen sein.

In einer dritten Variante werden das erste Werkstoffpulver und das zweite Werkstoffpulver in Form eines Grünlings, der das erste Werkstoffpulver und das zweite Werkstoffpulver umfasst, in die Form eingebracht. Dabei wird der Grünling aus einem Vorprozess erhalten. In diesem Zusammenhang ist die Pulvergemischansammlung durch den Grünling gebildet.

In einer vierten Variante werden das erste Werkstoffpulver und das zweite Werkstoffpulver in Form eines Weißlings, der das erste Werkstoffpulver und das zweite Werkstoffpulver umfasst, in die Form eingebracht. Dabei wird der Weißling aus einem Vorprozess erhalten. In dieser Variante ist die Pulvergemischansammlung durch den Weißling gebildet.

In allen Varianten umfasst das erste Werkstoffpulver und/oder das zweite Werkstoffpulver Nanopartikel und/oder Glaskeramik-Partikel. Das schließt wiederum neun Varianten ein. In einer Variante A umfasst das erste Werkstoffpulver Nanopartikel. In einer Variante B umfasst das zweite Werkstoffpulver Nanopartikel. In einer Variante C umfassen sowohl das erste Werkstoffpulver als auch das zweite Werkstoffpulver Nanopartikel. In einer Variante D umfasst das erste Werkstoffpulver Glaskeramik-Partikel. In einer Variante E umfasst das zweite Werkstoffpulver Glaskeramik-Partikel. In einer Variante F umfassen sowohl das erste Werkstoffpulver als auch das zweite Werkstoffpulver Glaskeramik-Partikel. In einer Variante G umfasst das erste Werkstoffpulver sowohl Nanopartikel als auch Glaskeramik-Partikel. In einer Variante H umfasst das zweite Werkstoffpulver sowohl Nanopartikel als auch Glaskeramik-Partikel. In einer Variante I umfassen sowohl das erste Werkstoffpulver als auch das zweite Werkstoffpulver sowohl Nanopartikel als auch Glaskeramik-Partikel.

Im Unterschied zum Stand der Technik bestehen also in keiner der vorstehenden Varianten A bis I sowohl das erste Werkstoffpulver als auch das zweite Werkstoffpulver aus Glaspulver. Dabei ist natürlich nicht ausgeschlossen, dass das erste Werkstoffpulver und/oder das zweite Werkstoffpulver auch Glaspulver umfasst. Die Verwendung von Glaskeramik-Partikeln und/oder Nanopartikeln hat den Effekt, dass die Herstellung des Glaskeramik-Rohlings vereinfacht wird. Nachdem Glaskeramik-Partikel bereits keimgebildete und/oder kristallisierte Abschnitte umfassen, sind diese bis zu einer gewissen Temperatur stabil, auch unter Druck. Eine vorzeitige Verdichtung kann somit ausgeschlossen werden. Auf diese Weise werden unerwünschte Gaseinschlüsse verhindert. Was die Nanopartikel anbelangt, so bieten diese in einem vorgegebenen Volumen eine verhältnismäßig große Oberfläche. Das ist für die Oberflächenkristallisation förderlich. Es können somit vergleichsweise schnell vergleichsweise Kristallstrukturen geschaffen werden. Die Glaskeramik-Partikel und/oder die Nanopartikel werden also so gewählt, dass sie den Herstellungsprozess erleichtern oder erst ermöglichen, da sie als Keimbildner für die spätere Kristallisation anderer Phasen dienen. Alternativ oder zusätzlich können die Glaskeramik-Partikel und/oder die Nanopartikel derart gewählt werden, dass sie die optischen und/oder mechanischen Eigenschaften des Glaskeramik-Rohlings positiv beeinflussen. Insbesondere können die Glaskeramik-Partikel und/oder die Nanopartikel dazu verwendet werden, einen erwünschten Farbverlauf und/oder einen erwünschten Transluzenzverlauf im Glaskeramik-Rohling zu bewirken. Ferner kann mittels der Glaskeramik-Partikel und/oder der Nanopartikel ein gewünschter Opaleszenzverlauf und/oder ein gewünschter Fluoreszenzverlauf geschaffen werden. Zu diesem Zweck müssen die Glaskeramik-Partikel und/oder die Nanopartikel entsprechende Opaleszenzeigenschaften und/oder entsprechende Fluoreszenzeigenschaften aufweisen. Auf diese Weise lassen sich die optischen Eigenschaften natürlicher Zähne besonders gut nachahmen.

Unter Nanopartikeln werden vorliegend Partikel mit einer Korngröße von 1nm bis 100nm verstanden.

Im Rahmen der vorliegenden Erfindung sind Glaskeramik-Partikel derart zu verstehen, dass sowohl Partikel aus keimgebildeter Glaskeramik als auch Partikel aus kristallisierter Glaskeramik umfasst sind.

Die Glaskeramik-Partikel haben eine Korngröße von mehr als 100nm und weniger als 2mm.

Gemäß einer Ausführungsform werden das erste Werkstoffpulver und das zweite Werkstoffpulver in sich lokal unterscheidenden Mischungsverhältnissen in die Form eingebracht, sodass sich ein Farbverlauf im Glaskeramik-Rohling ergibt. Es lässt sich auf diese Weise einfach und zuverlässig ein kontinuierlicher Farbverlauf im Glaskeramik-Rohling schaffen. Der Farbverlauf kann auch innerhalb des Glaskeramik-Rohlings lokal unterschiedlich sein. Aufgrund der Tatsache, dass Pulver in die Form eingebracht werden, kann der Farbverlauf sehr fein eingestellt werden.

In einer Variante wird die Pulvergemischansammlung wärmebehandelt. Das kann insbesondere dazu dienen, einen Bestandteil der Pulvergemischansammlung, der im Glaskeramik-Rohling unerwünscht ist, thermisch zu entfernen. Man spricht in diesem Zusammenhang auch von Ausbrennen. Beispielsweise können mittels einer derartigen Wärmebehandlung Bindemittel aus der Pulvergemischansammlung entfernt werden. Zudem kann die Wärmebehandlung ein Vorsintern bewirken.

Gemäß einer Alternative umfasst wenigstens eines aus erstem Werkstoffpulver und zweiten Werkstoffpulver verrundete Glaspartikel, verrundete Nanopartikel und/oder verrundete Glaskeramik-Partikel. In diesem Zusammenhang verbessert die Verrundung die Rieselfähigkeit der Glaspartikel, Nanopartikel und/oder Glaskeramik-Partikel. Das gilt insbesondere im Vergleich zu nicht-verrundeten Partikeln. Die Verrundung erleichtert dabei das Einfüllen des ersten Werkstoffpulvers und des zweiten Werkstoffpulvers in die Form. Das ist besonders hilfreich, wenn das erste Werkstoffpulver und das zweite Werkstoffpulver direkt in die Form eingefüllt werden. Es können also Vorprozesse zur Herstellung eines Grünlings und oder eines Weißlings entfallen. Dadurch wird das Herstellungsverfahren zur Herstellung des Glaskeramik-Rohlings vereinfacht.

Die Glaspartikel, Nanopartikel und/oder Glaskeramik-Partikel können beispielsweise mechanisch verrundet werden. In diesem Zusammenhang werden die Glaspartikel, Nanopartikel und/oder Glaskeramik-Partikel unter Nutzung von Mahlkörpern gemahlen. Alternativ oder zusätzlich können die Glaspartikel, Nanopartikel und/oder Glaskeramik-Partikel thermisch verrundet werden. Zu diesem Zweck können die Glaspartikel, Nanopartikel und/oder Glaskeramik-Partikel einer Plasma-Behandlung unterzogen werden.

Im Rahmen der vorliegenden Erfindung können als Glaspartikel Werkstoffsysteme zur Ausbildung von Glaskeramiken über das Schmelz-Giess-Verfahren verwendet werden, die für die Ausbildung von Lithiumdisilikat und/oder Lithiummetasilikat geeignet sind.

Als Glaskeramikpartikel können bereits kristallisierte Zusammensetzungen aus der Gruppe vorgenannter Glaspartikel in verschiedenen Kristallisationsstufen verwendet werden. Diese können optional Pigmente, z. B. Fluoreszenzpigmente wie Europium-dotiertes-Strontiumaluminat umfassen. Europium-dotiertes-Strontiumaluminat ist in der EP3696150A1 beschrieben, auf die in diesem Zusammenhang Bezug genommen wird.

Ferner können im Rahmen der vorliegenden Erfindung Nanopartikel Pigmente oder Trübungsmittel sein.

Ferner ist es möglich, dass Nanopartikel keramische Komponenten zur Einstellung von Bearbeitungseigenschaften als Störstellen im Gefüge umfassen. Beispiele hierfür sind Zirkoniumdioxid, Aluminiumoxid und Siliziumdioxid in verschiedenen Modifikationen.

Auch können Nanopartikel als Keimbildner für die heterogene Keimbildung (z.B. Metallkolloide, andere Fremdkristalle) ausgebildet sein.

Ferner ist es möglich, als Nanopartikel besonders kleine Partikel aus den Gruppen der Glaspartikel oder Glaskeramikpartikel zu verwenden. Dies dient der Erhöhung der Sinteraktivität.

Beim Verdichten der Pulvergemischansammlung mittels Heißpressen kann die Pulvergemischansammlung in der Form positioniert sein. Das heißt, dass das Heißpressen in derjenigen Form stattfindet, in die das erste Werkstoffpulver und das zweite Werkstoffpulvers zum Bilden der Pulvergemischansammlung eingebracht wurden. Es wird also zum Ausführen des erfindungsgemäßen Verfahrens lediglich eine einzige Form verwendet, in die das erste Werkstoffpulver und das zweite Werkstoffpulver eingebracht werden und die beim Heißpressen verwendet wird. Das Verfahren kann daher besonders effizient ablaufen.

Beim Verdichten der Pulvergemischansammlung mittels Heißpressen kann die Pulvergemischansammlung zuerst auf eine Temperatur von wenigstens 700°C erwärmt werden und danach eine Presskraft aufgebracht werden. Dabei wird durch das Erwärmen der Pulvergemischansammlung auf 700 °C erreicht, dass die Pulvergemischansammlung zuverlässig entgast. Erst danach wird die Presskraft aufgebracht. Auf diese Weise lassen sich qualitativ hochwertige Glaskeramik-Rohlinge herstellen. Das gilt insbesondere dann, wenn das erste Werkstoffpulver und das zweite Werkstoffpulver direkt in die Form eingebracht werden und Zwischenschritte zur Bildung eines Grünlings und/oder eines Weißlings fortgelassen werden.

Dabei kann das Erreichen der Temperatur von wenigstens 700°C als Auslösekriterium für das Aufbringen der Presskraft verwendet werden. Die Pulvergemischansammlung wird also hinsichtlich ihrer Temperatur überwacht. Dabei liegt die Erkenntnis zugrunde, dass beim Erreichen einer Temperatur von 700 °C die Pulvergemischansammlung ausreichend entgast ist.

Das Verfahren insgesamt und das Verdichten mittels Heißpressen im Speziellen lassen sich somit einfach und zuverlässig steuern.

Bevorzugt wird die Pulvergemischansammlung mittels Heißpressen auf eine Dichte von wenigstens 99,9% des Basismaterials des ersten Werkstoffpulvers und/oder des Basismaterials des zweiten Werkstoffpulvers verdichtet. Mit anderen Worten weist der Glaskeramik-Rohling nach dem Verdichten mittels Heißpressen eine Dichte auf, die im Wesentlichen der Dichte des Basismaterials des ersten Werkstoffpulvers und/oder des Basismaterials des zweiten Werkstoffpulvers entspricht. Auf diese Weise lassen sich Glaskeramik-Rohlinge von hoher mechanischer Qualität herstellen.

Gemäß einer Ausführungsform werden das erste Werkstoffpulver und das zweite Werkstoffpulver an wenigstens zwei voneinander separaten Stellen der Form in die Form eingebracht, um wenigstens zwei Pulvergemischansammlungen zu bilden. Mit einer solchen Form lassen sich folglich wenigstens zwei Glaskeramik-Rohlinge herstellen, aus jeder Pulvergemischansammlung einer. Eine solche Form kann auch als Mehrfachform bezeichnet werden, da unter Nutzung einer solchen Form zeitgleich mehrere Glaskeramik-Rohlinge hergestellt werden können. Auf diese Weise lassen sich in vergleichsweise kurzer Zeit vergleichsweise viele Glaskeramik-Rohlinge herstellen.

Allgemein gesprochen kann das erfindungsgemäße Verfahren also in Verbindung mit einer Einfachform, d.h. einer Form, die zur Herstellung eines einzigen Glaskeramik-Rohlings ausgebildet ist, oder in Verbindung mit einer Mehrfachform ausgeführt werden.

Das Verdichten der Pulvergemischansammlung mittels Heißpressen kann bei einer Temperatur von 650°C bis 980°C, insbesondere 700°C bis 750°C, und bei einem Druck von insbesondere 5 MPa bis 50 MPa, bevorzugt 10 MPa bis 30 MPa, erfolgen. Es entstehen somit Glaskeramik-Rohlinge von hoher mechanischer und optischer Qualität. Dabei werden Temperaturen vermieden, die materialspezifisch zu tief sind und somit entweder kein Verdichten erlauben oder zu unerwünschten Gaseinschlüssen führen. Ebenso werden materialspezifisch zu hohe Temperaturen vermieden, die zur Bildung unerwünschter Kristalle führen können.

Das Verdichten der Pulvergemischansammlung mittels Heißpressen kann für eine Dauer von 0,1 Minuten bis 10 Minuten, bevorzugt 0,3 Minuten bis 5 Minuten, erfolgen. Das Verdichten mittels Heißpressen benötigt also nur eine vergleichsweise kurze Zeit. Somit lässt sich der Glaskeramik-Rohling in vergleichsweise kurzer Zeit herstellen. Es versteht sich, dass dabei die optischen und mechanischen Anforderungen an den Glaskeramik-Rohling erfüllt werden.

Das Verdichten der Pulvergemischansammlung mittels Heißpressen kann bei einem Atmosphärendruck von weniger als 0,1 bar und bevorzugt von 0,01 bar bis 0,08 bar erfolgen. Vereinfacht gesagt findet das Verdichten der Pulvergemischansammlung mittels Heißpressen unter Vakuum statt. Auf diese Weise werden unerwünschte Reaktionen mit einer Umgebungsatmosphäre verringert oder ausgeschlossen. Auf diese Weise werden auch die Werkzeuge geschützt.

Gemäß einer Variante ist der Glaskeramik-Rohling ein Mehrfachrohling. Das bedeutet, dass der Glaskeramik-Rohling dazu ausgebildet ist, für zwei oder mehr dentale Restaurationen verwendet zu werden. Das impliziert, dass der Glaskeramik-Rohling vor, während oder nach dem Herstellen der dentalen Restaurationen in wenigstens zwei Stücke geteilt werden muss. Beispielsweise kann der Glaskeramik-Rohling zerschnitten oder zerbrochen werden. Das erfolgt bevorzugt vor dem Herstellen der dentalen Restauration. Auch kann der Rohling während oder nach dem Herstellen der dentalen Restauration innerhalb einer Fräs- oder Schleifmaschine getrennt werden. In diesem Fall erfolgt eine spanende Trennung. Durch das Herstellen von Mehrfachrohlingen lassen sich somit auf effiziente und zuverlässige Weise viele Glaskeramik-Rohlinge innerhalb von kurzer Zeit produzieren.

Das Verfahren kann auch ein Öffnen der Form und ein Entnehmen des Glaskeramik-Rohlings umfassen. Insbesondere wird dabei die Form geöffnet, solange sowohl die Form als auch der Glaskeramik-Rohling noch heiß sind. In diesem Zusammenhang spricht man auch vom Heißentformen oder vom Entformen im heißen Zustand. Mit anderen Worten werden die Form und der darin enthaltene Glaskeramik-Rohling nicht definiert abgekühlt, bevor die Form geöffnet und der Glaskeramik-Rohling entnommen wird. Auf diese Weise wird die Effizienz der Nutzung der Form gesteigert. Es versteht sich, dass der Glaskeramik-Rohling dann außerhalb der Form abkühlt.

Außerdem wir die Aufgabe durch einen mehrfarbigen Glaskeramik-Rohling gelöst, der mittels eines erfindungsgemäßen Verfahrens erhältlich ist. Wie bereits erläutert wurde, lässt sich ein derartiger Glaskeramik-Rohling effizient, was insbesondere kosteneffizient einschließt, herstellen. Dabei ist der Glaskeramik-Rohling unter mechanischen und optischen Gesichtspunkten hochwertig. Der Glaskeramik-Rohling kann also zu einer dentalen Restauration weiterverarbeitet werden. Insbesondere können aus einem derartigen Rohling Kronen, Abutments, Abutmentkronen, Inlays, Onlays, Veneers, Schalen, Brücken sowie Überwürfe hergestellt werden. Aufgrund der guten mechanischen und optischen Eigenschaften des Glaskeramik-Rohlings verfügen auch die dentalen Restaurationen über gute mechanische und optische Eigenschaften.

Ferner wird die Aufgabe durch eine Verwendung des erfindungsgemäßen mehrfarbigen Glaskeramik-Rohlings als Dentalmaterial gelöst. Insbesondere wird der erfindungsgemäße mehrfarbige Glaskeramik-Rohling zur Herstellung einer dentalen Restauration verwendet. Aufgrund der guten mechanischen und optischen Eigenschaften des Glaskeramik-Rohlings lassen sich auf diese Weise dentale Restaurationen mit guten mechanischen und optischen Eigenschaften herstellen.

Die Erfindung wird nachstehend anhand verschiedener Ausführungsbeispiele erläutert, die in den beigefügten Zeichnungen gezeigt sind. Es zeigen:
- Figur 1: einen erfindungsgemäßen, mehrfarbigen Glaskeramik-Rohling, der mittels eines erfindungsgemäßen Verfahrens hergestellt wurde, und seine Verwendung zur Herstellung einer dentalen Restauration,
- Figur 2: einen Ablauf eines erfindungsgemäßen Verfahrens zur Herstellung des mehrfarbigen Glaskeramik-Rohlings aus Figur 1,
- Figur 3: eine Variante des Verfahrens aus Figur 2, und
- Figur 4: eine weitere Variante des Verfahrens auf Figur 2.

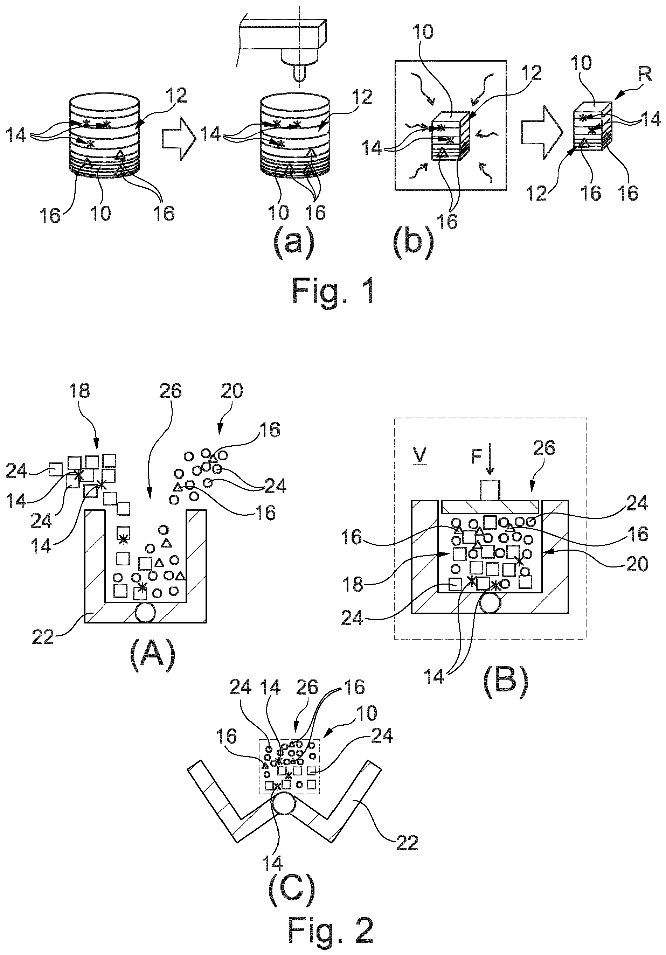

Figur 1 zeigt einen mehrfarbigen Glaskeramik-Rohling 10.

Der Glaskeramik-Rohling 10 umfasst eine Hauptphase 12 aus Glaskeramik, wobei sowohl Nanopartikel 14 als auch Glaskeramik-Partikel 16 in dieser Hauptphase 12 aus Glaskeramik eingebettet sind.

In der Darstellung in Figur 1 sind die Nanopartikel 14 und die Glaskeramik-Partikel 16 lediglich symbolisch und in übertriebener Größe illustriert.

Dabei unterscheiden sich die Glaskeramik-Partikel 16 dadurch von der Hauptphase 12 aus Glaskeramik, dass die Glaskeramik-Partikel 16 bei der Herstellung der Hauptphase 12 bereits vorlagen. Dies wird später noch im Detail erläutert werden.

Der Glaskeramik-Rohling 10, d. h. insbesondere die Hauptphase 12, ist dabei aus unterschiedlich gefärbten Werkstoffpulvern hergestellt, sodass der Glaskeramik-Rohling 10 einen kontinuierlichen Farbverlauf aufweist. Dies ist in Figur 1 durch die Schraffuren mit variierender Weite illustriert.

Der Glaskeramik-Rohling 10 wird als dentales Material zur Herstellung einer dentalen Restauration R verwendet.

In diesem Zusammenhang wird der Glaskeramik-Rohling 10 im Schritt (a) spanend bearbeitet, sodass der Glaskeramik-Rohling 10 die Form der herzustellenden dentalen Restauration R erhält. Es versteht sich dabei, dass die dentale Restauration in Figur 1 lediglich schematisch dargestellt ist.

In einem auf den Schritt (a) folgenden Schritt (b) wird der spanend bearbeitete Glaskeramik-Rohling 10 thermisch gehärtet. In diesem Zusammenhang finden innerhalb des Glaskeramik-Rohlings 10 Kristallisationsvorgänge statt. Es werden also die mechanischen Eigenschaften des Glaskeramik-Rohlings 10 eingestellt.

Anschließend kann die auf diese Weise hergestellte dentale Restauration R an einem Patienten verwendet werden.

Der Glaskeramik-Rohling 10 wird mittels eines Verfahrens hergestellt, das im Folgenden anhand der Figuren 2, 3 und 4 erläutert werden wird.

Im Beispiel gemäß Figur 2 werden in einem ersten Verfahrensschritt A ein erstes Werkstoffpulver 18 sowie ein zweites Werkstoffpulver 20 in einer Form 22 eingebracht.

Das erste Werkstoffpulver 18 und das zweite Werkstoffpulver 20 sind dabei unterschiedlich gefärbt.

Darüber hinaus umfasst das erste Werkstoffpulver 18 Nanopartikel 14 und Glaspartikel 24.

Das zweite Werkstoffpulver 20 umfasst Glaskeramik-Partikel 16 und Glaspartikel 24.

Dabei sind die Glaspartikel 24, die Nanopartikel 14 und die Glaskeramik-Partikel 16 verrundet.

Ferner werden das erste Werkstoffpulver 18 und das zweite Werkstoffpulver 20 in lokal unterschiedlichen Mischungsverhältnissen in die Form 22 eingebracht.

Es versteht sich, dass in Figur 2 die Glaspartikel 24, die Nanopartikel 144 und die GlaskeramikPartikel 16 lediglich schematisch und stark vergrößert dargestellt sind. Um die unterschiedliche Färbung zu symbolisieren, sind die Glaspartikel 24 des ersten Werkstoffpulvers 18 als Vierecke dargestellt und die Glaspartikel 24 des zweiten Werkstoffpulvers 20 als Kreise. Der besseren Übersichtlichkeit wegen sind nur einige Partikel mit einem Bezugszeichen versehen.

Im dargestellten Ausführungsbeispiele nimmt in einem unteren Bereich der Form 22 das erste Werkstoffpulver 18 einen größeren Anteil ein als das zweite Werkstoffpulver 20. In einem oberen Bereich der Form 22 ist es umgekehrt.

Nachdem das erste Werkstoffpulver 18 und das zweite Werkstoffpulver 20 unterschiedlich gefärbt sind, ergibt sich auf diese Weise ein Farbverlauf innerhalb der Pulvergemischansammlung 26, die durch das Einbringen des ersten Werkstoffpulvers 18 und des zweiten Werkstoffpulvers 20 in der Form 22 gebildet wird. Dieser Farbverlauf bleibt im fertigen Glaskeramik-Rohling 10 bestehen.

Innerhalb der Form 22 kann die Pulvergemischansammlung 26 optional zu einem Grünling verdichtet werden.

Weiter optional kann die Pulvergemischansammlung 26 wärmebehandelt werden.

In einem nachfolgenden Verfahrensschritt B wird die Pulvergemischansammlung 26 mittels Heißpressen verdichtet. Auf diese Weise wird aus der Pulvergemischansammlung 26 der Glaskeramik-Rohling 10 gebildet.

Die Pulvergemischansammlung 26 bleibt hierzu in der Form 22.

Im Detail wird im Zuge des Heißpressens zunächst die Pulvergemischansammlung 26 auf eine Temperatur von 700 °C erwärmt. Erst wenn die Pulvergemischansammlung 26 die Temperatur von 700 °C erreicht, wird eine Presskraft F auf die Pulvergemischansammlung 26 aufgebracht. Mit anderen Worten stellt das Erreichen der Temperatur von 700 °C das Auslösekriterium für das Aufbringen der Presskraft F dar.

Die Presskraft F ist dabei so gewählt, dass die Pulvergemischansammlung 26 einem Druck von 10 MPa bis 30 MPa ausgesetzt ist. Vorliegend beträgt der Druck 20MPa.

Zudem wird die Temperatur der Pulvergemischansammlung 26 während des Aufbringens der Presskraft F weiter erhöht. Vorliegend wird die Temperatur auf 730 °C erhöht.

Außerdem findet das Verdichten in einer Vakuumkammer V statt. Innerhalb der Vakuumkammer V herrscht ein Druck von 0,01 bis 0,08 bar.

Im vorliegenden Ausführungsbeispiel werden der genannte Druck und die genannte Temperatur für eine Dauer von 4 Minuten aufrechterhalten.

Danach wird in einem Verfahrensschritt C die Form 22 geöffnet und der Glaskeramik-Rohling 10 wird aus der Form 22 entnommen. Dies erfolgt im Wesentlichen direkt nach dem Verfahrensschritt B. Es findet also kein definierter Abkühlvorgang statt.

Es versteht sich, dass die im Verfahrensschritt C dargestellte Öffnung der Form 22 lediglich schematisch ist und die Form 22 auf jede andere geeignete Weise geöffnet werden kann.

Der auf diese Weise hergestellte Glaskeramik-Rohling 10 erreicht eine Dichte von 99,9 % des Basismaterials des ersten Werkstoffpulvers 18.

Was die geometrischen Dimensionen des Glaskeramik-Rohlings 10 anbelangt, so ist dieser derart dimensioniert, das mittels eines bereits im Zusammenhang mit Figur 1 erläuterten Verfahrens eine einzige dentale Restauration R aus dem Glaskeramik-Rohling 10 herausgearbeitet werden kann.

Der Glaskeramik-Rohling 10 kann dabei als Kronen-Rohling, Inlay-Rohling oder Brücken-Rohling ausgebildet sein. Derartige Rohlinge und zugehörige Abmessungen sind allgemein bekannt.

Beispielsweise hat ein Kronen-Rohling Abmessungen von 18,4mm x 14,7mm x 12,5mm. Ein Brücken-Rohling kann Abmessungen von 15mm x 32mm x 15mm haben.

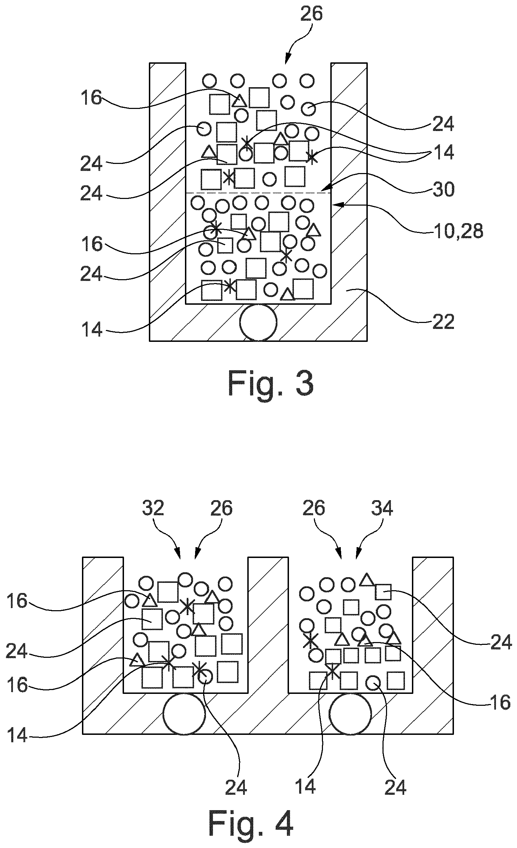

In Figur 3 ist eine Variante des in Figur 2 dargestellten Verfahrens illustriert.

Dabei wird lediglich auf die Unterschiede gegenüber dem Verfahren aus Figur 2 eingegangen. Gleiche oder einander entsprechende Elemente sind mit selben Bezugszeichen versehen.

In der Variante gemäß Figur 3 wird eine Form 22 verwendet, deren Inneres im Wesentlichen doppelt so groß ist wie bei der Form 22, die Verfahren gemäß Figur 2 verwendet wird.

Es lässt sich somit in der Variante gemäß Figur 3 ein Glaskeramik-Rohling 10 herstellen, der im Wesentlichen doppelt so groß ist wie der Glaskeramik-Rohling 10, der mittels des Verfahrens aus Figur 2 hergestellt werden kann.

Dementsprechend ist der Glaskeramik-Rohling 10, der mit dem Verfahren gemäß Figur 3 hergestellt wird, so dimensioniert, das mittels eines im Zusammenhang mit Figur 1 erläuterten Verfahrens zwei dentale Restaurationen R aus dem Glaskeramik-Rohling 10 herausgearbeitet werden können.

Es versteht sich dabei, dass auch Glaskeramik-Rohlinge 10 denkbar sind, die zur Herstellung von mehr als zwei dentalen Restaurationen R geeignet sind.

Der Glaskeramik-Rohling 10, der mittels der Variante aus Figur 3 hergestellt werden kann, kann daher auch als Mehrfachrohling 28 bezeichnet werden.

Innerhalb des Mehrfachrohlings 28 kann eine Trennebene 30 vorgesehen sein. Wenn der Mehrfachrohling 28 entlang dieser Trennebene 30 zertrennt wird, ergeben sich zwei Glaskeramik-Rohlinge 10, die in ihren Dimensionen einem Glaskeramik-Rohling 10 entsprechen, der mittels des Verfahrens gemäß Figur 2 erhältlich ist.

Eine weitere Variante des Verfahrens zur Herstellung eines Glaskeramik-Rohlings 10 ist in Figur 4 illustriert.

Wieder wird lediglich auf die Unterschiede gegenüber dem Verfahren gemäß Figur 2 eingegangen. Gleiche oder einander entsprechende Elemente sind mit denselben Bezugszeichen versehen.

Die Unterschiede betreffen wieder die verwendete Form 22.

Im Verfahren gemäß Figur 4 weist die Form 22 zwei Kavitäten 32, 34 auf, wobei in jeder der Kavitäten 32, 34 eine Pulvergemischansammlung 26 zur Herstellung eines Glaskeramik-Rohlings 10 aufgenommen werden kann. Mit anderen Worten können mittels einer derartigen Form 22 gleichzeitig zwei Glaskeramik-Rohlinge 10 hergestellt werden, die in ihren Dimensionen dem Glaskeramik-Rohling 10 entsprechen, der mittels des Verfahrens gemäß Figur 2 erhältlich ist.

Dementsprechend werden in der Variante gemäß Figur 4 im Verfahrensschritt A an wenigstens zwei voneinander separaten Stellen der Form 22, die den Kavitäten 32, 34 entsprechen, das erste Werkstoffpulver 18 und das zweite Werkstoffpulver 20 in die Form 22 eingebracht. Es werden somit zwei voneinander separate Pulvergemischansammlung 26 gebildet.

Eine derartige Form 22 kann auch als Mehrfachform 36 bezeichnet werden.

Die vorstehenden Beispiele können auch kombiniert werden. In diesem Zusammenhang ist beispielsweise eine Variante denkbar, bei der das Verfahren mit einer Mehrfachform ausgeführt wird, die dazu ausgebildet ist, mehrere Mehrfachrohlinge herzustellen. Auf diese Weise lassen sich gleichzeitig besonders viele Glaskeramik-Rohlinge herstellen.

### Bezugszeichenliste

- 10: Glaskeramik-Rohling
- 12: Hauptphase
- 14: Nanopartikel
- 16: Glaskeramik-Partikel
- 18: erstes Werkstoffpulver
- 20: zweites Werkstoffpulver
- 22: Form
- 24: Glaspartikel
- 26: Pulvergemischansammlung
- 28: Mehrfachrohling
- 30: Trennebene
- 32: Kavität
- 34: Kavität
- 36: Mehrfachform

- F: Presskraft
- R: dentale Restauration
- V: Vakuumkammer

## Patentansprüche

1. Verfahren zur Herstellung eines mehrfarbigen Glaskeramik-Rohlings (10) für dentale Zwecke, aus wenigstens einem ersten Werkstoffpulver (18) und einem zweiten Werkstoffpulver (20), wobei das erste Werkstoffpulver (18) und das zweite Werkstoffpulver (20) unterschiedlich gefärbt sind und wobei wenigstens eines aus erstem Werkstoffpulver (18) und zweitem Werkstoffpulver (20) Nanopartikel (14) und/oder Glaskeramik-Partikel (16) umfasst, wobei das Verfahren umfasst:
- Einbringen des ersten Werkstoffpulvers (18) und des zweiten Werkstoffpulvers (20) in eine Form (22), um wenigstens eine Pulvergemischansammlung (26) zu bilden, und
- Verdichten der Pulvergemischansammlung (26) mittels Heißpressen, um den Glaskeramik-Rohling (10) zu bilden.

2. Verfahren nach Anspruch 1, wobei das erste Werkstoffpulver (18) und das zweite Werkstoffpulver (20) in sich lokal unterscheidenden Mischungsverhältnissen in die Form (22) eingebracht werden, sodass sich ein Farbverlauf im Glaskeramik-Rohling (10) ergibt.

3. Verfahren nach Anspruch 1 oder 2, ferner umfassend Wärmebehandeln der Pulvergemischansammlung (26).

4. Verfahren nach einem der vorhergehenden Ansprüche, wobei wenigstens eines aus erstem Werkstoffpulver (18) und zweitem Werkstoffpulver (20) verrundete Glaspartikel (24), verrundete Nanopartikel (14) und/oder verrundete Glaskeramik-Partikel (16) umfasst.

5. Verfahren nach einem der vorhergehenden Ansprüche, wobei beim Verdichten der Pulvergemischansammlung (26) mittels Heißpressen die Pulvergemischansammlung (26) in der Form (22) positioniert ist.

6. Verfahren nach einem der vorhergehenden Ansprüche, wobei beim Verdichten der Pulvergemischansammlung (26) mittels Heißpressen die Pulvergemischansammlung (26) zuerst auf eine Temperatur von wenigstens 700°C erwärmt wird und danach eine Presskraft (F) aufgebracht wird.

7. Verfahren nach Anspruch 6, wobei das Erreichen der Temperatur von wenigstens 700°C als Auslösekriterium für das Aufbringen der Presskraft (F) verwendet wird.

8. Verfahren nach einem der vorhergehenden Ansprüche, wobei die Pulvergemischansammlung (26) mittels Heißpressen auf eine Dichte von wenigstens 99,9% des Basismaterials des ersten Werkstoffpulvers (18) und/oder des Basismaterials des zweiten Werkstoffpulvers (20) verdichtet wird.

9. Verfahren nach einem der vorhergehenden Ansprüche, wobei an wenigstens zwei voneinander separaten Stellen der Form (22) das erste Werkstoffpulver (18) und das zweite Werkstoffpulver (20) in die Form (22) eingebracht werden, um wenigstens zwei Pulvergemischansammlungen (26) zu bilden.

10. Verfahren nach einem der vorhergehenden Ansprüche, wobei das Verdichten der Pulvergemischansammlung (26) mittels Heißpressen bei einer Temperatur von 650°C bis 780°C, insbesondere 700°C bis 750°C, und bei einem Druck von insbesondere 5 MPa bis 50 MPa, bevorzugt 10 MPa bis 30 MPa, erfolgt.

11. Verfahren einem der vorhergehenden Ansprüche, wobei das Verdichten der Pulvergemischansammlung (26) mittels Heißpressen für eine Dauer von 0,1 Minuten bis 10 Minuten, bevorzugt 0,3 Minuten bis 5 Minuten, erfolgt.

12. Verfahren nach einem der vorhergehenden Ansprüche, wobei das Verdichten der Pulvergemischansammlung (26) mittels Heißpressen bei einem Atmosphärendruck von weniger als 0,1 bar und bevorzugt von 0,01 bar bis 0,08 bar erfolgt.

13. Verfahren nach einem der vorhergehenden Ansprüche, wobei der Glaskeramik-Rohling (10) ein Mehrfachrohling (28) ist.

14. Verfahren nach einem der vorhergehenden Ansprüche, ferner umfassend Öffnen der Form (22) und Entnehmen des Glaskeramik-Rohlings (10).

15. Mehrfarbiger Glaskeramik-Rohling (10) erhältlich mittels eines Verfahrens nach einem der vorhergehenden Ansprüche.

16. Verwendung des mehrfarbigen Glaskeramik-Rohlings (10) nach Anspruch 15 als Dentalmaterial, insbesondere zur Herstellung einer dentalen Restauration (R).
